# EUROPEAN PATENT SPECIFICATION

(11) **EP 2 793 954 B1**
(45) Date of publication and mention of the grant of the patent: **21.10.2020**
(21) Application number: 12806500.0
(22) Date of filing: 21.12.2012
(51) Int. Cl.: A61K 51/04, A61K 47/12, C07B 59/00, A61K 101/02

(54) **18F-FLUCICLOVINE COMPOSITIONS IN CITRATE BUFFERS**
18F-FLUCICLOVINZUSAMMENSETZUNGEN IN CITRATPUFFERN
COMPOSITIONS DE 18F-FLUCICLOVINE DANS DES TAMPONS CITRATE

(30) Priority: 21.12.2011 WO PCT/EP2011/073670
(43) Date of publication of application: 29.10.2014
(60) Divisional application: 20196030.9
(73) Proprietor: GE Healthcare Limited, Chalfont St. Giles, Buckinghamshire HP8 4SP (GB)
(72) Inventor: ROMOREN, Kristine, 0401 Oslo (NO); RYAN, Olav, 0411 Olso (NO)
(74) Representative: Bannan, Sally
(86) International application number: PCT/EP2012/076689
(87) International publication number: WO 2013/093025

(56) References cited:
- EP-A1- 2 119 458
- Anders Svadberg ET AL: "Degradation of acetonitrile in eluent solutions for [18F]fluoride PET chemistry: impact on radiosynthesis of [18F]FACBC and [18F]FDG", Journal of Labelled Compounds and Radiopharmaceuticals, vol. 55, no. 3, 5 March 2012 (2012-03-05), pages 97-102, XP55079899, ISSN: 0362-4803, DOI: 10.1002/jlcr.1956

## Description

### Technical Field of the Invention

The present invention relates to a drug product composition and in particular to a composition comprising a positron emission tomography (PET) tracer. The composition of the present invention has certain advantages over prior art formulations.

### Description of Related Art

The non-natural amino acid [¹⁸F]-1-amino-3-fluorocyclobutane-1-carboxylic acid ([¹⁸F]FACBC, also known as [¹⁸F]-Fluciclovine) is taken up specifically by amino acid transporters and has shown promise for tumour imaging with positron emission tomography (PET).

In radioactive diagnostic imaging agents, a problem often arises such that compounds decompose by self-radiation during delivery of the agents so as to cause decrease in radiochemical purity due to so-called radiolysis. In PET tracers comprising nuclides such as ¹¹C and ¹⁸F, radiolysis often becomes more problematic since the half-life of the nuclides used therein is relatively short, e.g. as compared with nuclides used in single photon emission tomography (SPECT) such as ^{99m}Tc, and thus radioactivity upon shipment must be set larger than SPECT agents, thereby making the resulting radiation energy thereof higher.

Various methods for inhibiting radiolysis in PET tracers have been examined. For example in compositions comprising [¹⁸F]-fluorodeoxyglucose ([¹⁸F]FDG). WO 2003/090789 discloses a method of reducing the radiolysis of [¹⁸F]FDG by adding a weak acid-based buffer to an [¹⁸F]FDG solution. WO 2004/043497 discloses adding ethanol to a [¹⁸F]FDG solution to obtain a composition of [¹⁸F]-FDG having improved stability.

In the case of [¹⁸F]FACBC different strategies have been adopted. EP 2106808 (A1) discloses that for a composition comprising [¹⁸F]FACBC, when the pH value is not more than 5.9, stability thereof is maintained even if there exist no pharmaceutical additives or buffers that prevent radiolysis.

EP 2080526 (A1) discloses that radiolysis can be inhibited by adding a sugar lactone such as ascorbic acid and glucono-o-lactone to [¹⁸F]FACBC. An exemplary composition taught by EP 2080526 (A1) has a radioactivity of 1.4GBq in about 2 mL and contains the sugar lactone in a proportion of 10mmol/mL immediately after production providing a radioactivity of 50 to 225 MBq when the agent is used, sufficient for PET imaging in adults. It was also disclosed that ascorbic acid at concentrations of 0.5-10.0 µmol/mL can inhibit decomposition of [¹⁸F]FACBC solution. In this case, radiolysis was inhibited at a concentration of 700 MBq/mL at maximum.

EP 2119458 (A1) discloses a method to prepare a stabilised formulation of [¹⁸F]FACBC comprising diluting a solution of [¹⁸F]FACBC and then adding an acid in an amount sufficient to adjust the pH of the solution to 2.0-5.9. Suitable acids disclosed are ascorbic acid, benzoic acid, hydrochloric acid, acetic acid, citric acid, gentisic acid, and oxalic acid, with hydrochloric acid preferred. EP 2119458 (A1) also discloses that a sugar alcohol such as erythritol xylitol, sorbitol or mannitol can be added as a further additive to inhibit radiolysis and improve stability.

In these known [¹⁸F]FACBC compositions radiostability is maintained by adjusting pH within a relatively wide range using an acid and/or including a suitable additive. Adjustment of pH using an acid rather than using a buffer has the advantage that the ionic strength of the composition is lower.

### Summary of the Invention

The present invention provides a pharmaceutical composition comprising [¹⁸F]FACBC having certain advantages over known compositions comprising [¹⁸F]FACBC. Also provided by the present invention is a method to obtain the composition of the invention. The composition of the present invention is resistant to degradation, can be autoclaved or diluted in saline (i.e. 0.9 % NaCl), and still maintain its pH in a narrow range. Furthermore, the pharmaceutical composition of the present invention does not require any radiostabiliser in order to maintain good radiostability over its shelf-life.

### Detailed Description of the Invention

The present invention in one aspect provides a pharmaceutical composition of ¹⁸F-FACBC characterised in that said composition:
(i) comprises 50-100 mM citrate buffer; and,
(ii) has a pH of 4.0-5.0,
with the proviso that said composition does not comprise a sugar lactone or a sugar alcohol radiostabiliser.

The term "pharmaceutical composition" refers to a composition comprising a pharmaceutical together with a biocompatible carrier in a form suitable for mammalian administration. A "biocompatible carrier" is a fluid, especially a liquid, in which a pharmaceutical is suspended or dissolved, such that the composition is physiologically tolerable, i.e. can be administered to the mammalian body without toxicity or undue discomfort. The biocompatible carrier is suitably an injectable carrier liquid such as sterile, pyrogen-free water for injection or an aqueous solution such as saline.

The pharmaceutical composition of the invention preferably 60-90 mM citrate buffer, most preferably 75-85 mM citrate buffer.

The pharmaceutical composition of the invention preferably has a pH of 4.1-4.5, most preferably 4.3-4.4.

The pharmaceutical composition of the invention preferably has an end of synthesis (EOS) radioactive concentration (RAC) of at least 1000 MBq/mL, alternatively at least 1500 MBq/ml.

The term "end of synthesis" refers to the point in time when the labelled compound is collected in the product collection vial.

The pharmaceutical composition of the present invention has a favourable impurity profile, with the main non-radioactive impurities being 1-amino-3-hydroxyl-cyclobutane-1-carboxylic acid (hydroxyl-ACBC), 1-amino-3-fluoro-cyclobutane-1-carboxylic acid (FACBC) and 1-amino-3-chloro-cyclobutane-1-carboxylic acid (chloro-ACBC).

It is preferred that there is not more than 150 µg/mL hydroxyl-ACBC, most preferably not more than 80 µg/mL hydroxyl-ACBC.

It is preferred that there is not more than 0.15 µg/mL FACBC, most preferably not more than 0.10 µg/mL FACBC.

It is preferred that there is not more than 2.0 µg/mL chloro-ACBC, most preferably not more than 1.0 µg/mL chloro-ACBC.

The term "not more than" should be understood to mean any amount less than the quoted quantity. Therefore not more than 100 µg/mL means any amount between 0-100 µg/mL, and in an *ideal* embodiment of the composition of the present invention there would be zero µg/mL of each impurity present in the composition of the invention. However, in reality, zero µg/mL of an impurity might not be achievable and it is more likely that at least a trace amount of the impurity remains in the composition, i.e. in the case of hydroxyl-ACBC the term not more than 150 µg/mL covers e.g. 50-150 µg/mL, not more than 0.10 µglmL for FACBC covers e.g. 0.05-0.10 µg/mL, and not more than 1.0 µg/mL chloro-ACBC covers e.g. 0.25-1.0 µg/mL.

An advantage of the composition of the present invention is that the pH, stability and impurity profile can be kept within a very narrow range over a long shelf-life, at high activities, and when manipulated e.g. by autoclaving or by dilution with 0.9% saline.

The pharmaceutical composition of the invention does not comprise a radiostabiliser being a sugar lactone or a sugar alcohol. It is common for pharmaceutical compositions comprising radioactive pharmaceuticals to include a radiostabiliser. For example, known pharmaceutical compositions of [¹⁸F]FACBC include a sugar alcohol or a sugar lactone.

EP 2080526 (A1) discloses that radiolysis can be inhibited by adding a sugar lactone such as ascorbic acid and glucono-o-lactone to [¹⁸F]FACBC, and EP 2119458 (A1) discloses that a sugar alcohol such as erythritol xylitol, sorbitol or mannitol can be added as an additive to inhibit radiolysis and improve stability. No such radiostabiliser is required in the radiopharmaceutical composition of the present invention in order to maintain a shelf-life of up to around 10 hours.

In another aspect the present invention provides a method to obtain a radiopharmaceutial composition wherein said composition is as defined hereinabove, and wherein said method comprises:
(i) reacting with a suitable source of [¹⁸F]fluoride a precursor compound of Formula I: 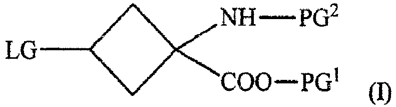 wherein.
   LG is a leaving group;
   PG¹ is a carboxy protecting group; and,
   PG² is an amine protecting group;
   to obtain a compound of Formula II: 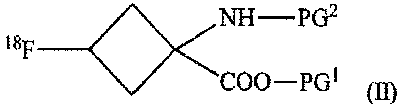 wherein PG¹ and PG² are as defined for Formula II;
(ii) reacting said compound of Formula II with a PG¹ deprotecting agent to obtain a compound of Formula III: 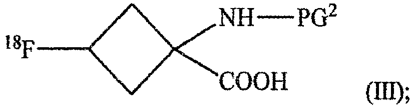 wherein PG² is as defined for Formula I;
(iii) reacting said compound of Formula III with a PG² deprotecting agent to obtain [¹⁸F]FACBC;
(iv) formulating said [¹⁸F]FACBC with citrate buffer to obtain said pharmaceutical composition.

The "source of [¹⁸F]fluoride" suitable for use in the invention is normally obtained as an aqueous solution from the nuclear reaction ¹⁸O(p,n)¹⁸F. In order to increase the reactivity of fluoride and to reduce or minimise hydroxylated by-products resulting from the presence of water, water is typically removed from [¹⁸F]-fluoride prior to the reaction, and fluorination reactions are carried out using anhydrous reaction solvents (Aigbirhio et al 1995 J Fluor Chem; 70: 279-87). A further step that is used to improve the reactivity of [¹⁸F]-fluoride for radiofluorination reactions is to add a cationic counterion prior to the removal of water. Suitably, the counterion should possess sufficient solubility within the anhydrous reaction solvent to maintain the solubility of the [¹⁸F]-fluoiide. Therefore, counterions that are typically used include large but soft metal ions such as rubidium or caesium, potassium complexed with a cryptand such as Kryptofix™, or tetraalkylammonium salts, wherein potassium complexed with a cryptand such as Kryptofix™, or tetraalkylammonium salts are preferred.

A "precursor compound" comprises a non-radioactive derivative of a radiolabelled compound, designed so that chemical reaction with a convenient chemical form of the detectable label occurs site-specifically; can be conducted in the minimum number of steps (ideally a single step); and without the need for significant purification (ideally no further purification), to give the desired radiolabelled compound. Such precursor compounds are synthetic and can conveniently be obtained in good chemical purity.

A suitable "leaving group" in the context of the present invention is a chemical group that can be displaced by nucleophilic displacement reaction with fluoride ion. These are well-known in the art of synthetic chemistry. In some embodiments the leaving group of the present invention is a linear or branched C₁₋₁₀ haloalkyl sulfonic acid substituent, a linear or branched C₁₋₁₀ alkyl sulfonic acid substituent, a fluorosulfonic acid substituent, or an aromatic sulfonic acid substituent. In other embodiments of the invention the leaving group is selected from methanesulfonic acid, toluenesulfonic acid, nitrobenzenesulfonic acid, benzenesulfonic acid, trifluoromethanesulfonic acid, fluorosulfonic acid, and perfluoroalkylsulfonic acid. In some embodiments the leaving group is either methanesulfonic acid, trifluoromethanesulfonic acid or toluenesulfonic acid and in another embodiment the leaving group is trifluoromethanesulfonic acid.

The term "protecting group" refers to a group which inhibits or suppresses undesirable chemical reactions, but which is designed to be sufficiently reactive that it may be cleaved from the functional group in question to obtain the desired product under mild enough conditions that do not modify the rest of the molecule. Protecting groups are well known to those skilled in the art and are described in 'Protective Groups in Organic Synthesis', Theorodora W. Greene and Peter G. M. Wuts, (Fourth Edition, John Wiley & Sons, 2007).

The PG¹ "carboxy protecting group" is preferably linear or branched C₁₋₁₀ alkyl chain or an aryl substituent. The term "alkyl" used either alone or as part of another group is defined as any straight, branched or cyclic, saturated or unsaturated CₙH₂ₙ₊₁ group. The term "aryl" refers to any C₆₋₁₄ molecular fragment or group which is derived from a monocyclic or polycyclic aromatic hydrocarbon, or a monocyclic or polycyclic heteroaromatic hydrocarbon. In one embodiment of the method of the invention PG¹ is selected from methyl, ethyl, t-butyl and phenyl. In another embodiment of the invention PG¹ is methyl or ethyl and in yet another embodiment PG¹ is ethyl.

The PG² "amine protecting group" suitably prevents reaction between ¹⁸F and the amino group in the process of providing the compound of Formula II. Examples of suitable amine protecting groups include various carbamate substituents, various amide substituents, various imide substituents, and various amine substituents. Preferably, the amine protecting group is selected from the group consisting of linear or branched C₂₋₇ alkyloxycarbonyl substituents, linear or branched C₃₋₇ alkenyloxycarbonyl substituents, C₇₋₁₂ benzyloxycarbonyl substituents that may have a modifying group, C₂₋₇ alkyldithiooxycarbonyl substituents, linear or branched C₁₋₆ alkylamide substituents, linear or branched C₂₋₆ amide substituents, C₆₋₁₁ benzamide substituents that may have a modifying group, C₄₋₁₀ cyclic imide substituents, C₆₋₁₁ aromatic imine substituents that may have a substituent, linear or branched C₁₋₆ alkylamine substituents, linear or branched C₂₋₆ alkenylamine substituents, and C₆₋₁₁ benzylamine substituents that may have a modifying group. In some embodiments of the invention PG² is selected from t-butoxycarbonyl, allyloxycarbonyl, phthalimide, and N-benzylideneamine. In other embodiments PG² is selected from t-butoxycarbonyl or phthalimide. In one embodiment of the invention PG² is t-butoxycarbonyl.

The term "reacting" refers to bringing two or more chemical substances (typically referred to in the art as "reactants" or "reagents") together to result in a chemical change in one or both/all of the chemical substances.

A "PG¹ deprotecting agent" is a reagent capable of removing the carboxy protecting group PG¹ from the compound of Formula II during the reacting step (b). Suitable such carboxy deprotecting agents are well-known to the skilled person (see Greene and Wuts, *supra*) and may be either an acid or an alkaline solution. The concentration of the PG¹ deprotecting agent is not limited as long as it is sufficient to remove the carboxy protecting group PG¹ and does not have an effect on the final purity or results in an incompatibility with any container used. Preferably the PG¹ deprotecting agent is an alkaline solution. In certain embodiments the PG¹ deprotecting agent is a sodium hydroxide or a potassium hydroxide solution and in a preferred embodiment is a sodium hydroxide solution, for example of 0.5-2.0M. The reacting step is enabled by closing the outlet of the SPE column so that the PG¹ deprotecting agent is retained therein for a specified amount of time. The temperature and the duration of this reacting step need to be sufficient to permit removal of the PG¹ carboxy deprotecting group. In certain embodiments the reacting step is carried out at room temperature and for a duration of between 1-5 minutes.

The "PG² deprotecting agent" is a reagent capable of removing the amine protecting group PG² from the compound of Formula III during the reacting step (e). Suitable such amine deprotecting agents are well-known to the skilled person (see Greene and Wuts, *supra*) and may be either an acid or an alkaline solution. The concentration of the PG² deprotecting agent is not limited as long as it is sufficient to remove the carboxy protecting group PG². Preferably the PG² deprotecting agent is an acid solution. A suitable acid preferably includes an acid selected from inorganic acids such as hydrochloric acid, sulfuric acid and nitric acid, and organic acids such as perfluoroalkyl carboxylic acid, e.g. trifluoroacetic acid. In certain embodiments, the PG² deprotecting agent is hydrochloric acid, and in other embodiments when HCl is used as PG² deprotecting agent it is at a concentration of 1.0-4.0M. Reacting step (e) is preferably carried out with heat to allow the removal of PG² reaction to proceed more rapidly. The reaction time depends on the reaction temperature or other conditions. For example, when the reacting step (e) is performed at 60°C, a sufficient reaction time is 5 minutes.

Precursor compounds of Formula I may be obtained by following or adapting methods known in the art, such as for example described by McConathy et al (2003 Appl Radiat Isotop; 58: 657-666) or by Shoup and Goodman (1999 J Label Comp Radiopharm; 42: 215-225).

In a preferred aspect, the [¹⁸F]-FACBC is trans-1-amino-3-[¹⁸F]-fluorocyclobutanecarboxylic acid (*anti*-(¹⁸F]-FACBC): 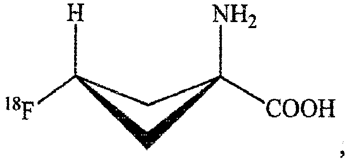 said compound of Formula I is a compound of Formula la: 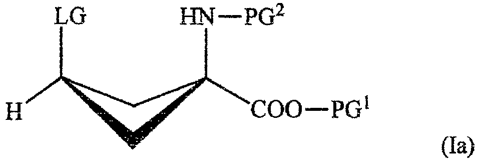 said compound of Formula II is a compound of Formula IIa: 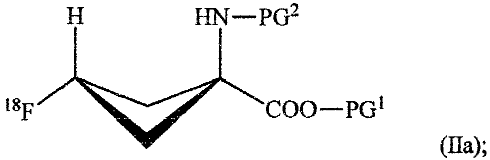 and,
said compound of Formula III is a compound of Formula IIIa: 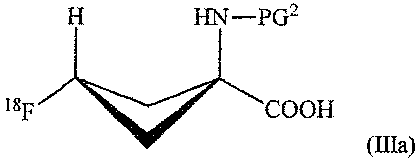 wherein PG¹ and PG² are as described hereinabove.

In some embodiments the method of present invention additionally includes a step following the reacting step and before the formulating step of purifying the reaction mixture obtained in the reacting step to obtain substantially pure [¹⁸F]FACBC.

The term "substantially" as used in "substantially pure" takes the meaning as presented above. The term "substantially pure" as used in the context of [¹⁸F]FACBC encompasses completely pure (¹⁸F]FACBC or [¹⁸F]FACBC that is sufficiently pure to be suitable for use as a PET tracer. The term "suitable for use as a PET tracer" means that the [¹⁸F]FACBC product is suitable for intravenous administration to a mammalian subject followed by PET imaging to obtain one or more clinically-useful images of the location and/or distribution of [¹⁸F]-FACBC.

A suitable purifying step comprises:
(i) carrying out a first purification step comprising passing said reaction mixture through a hydrophilic lipophilic balanced (HLB) solid phase; and,
(ii) optionally carrying out a second purification step comprising passing said reaction mixture through an alumina solid phase.

In certain embodiments of the present invention said purifying step can be said to consist essentially of the above-defined steps. In particular, the purifying step does not require that the reaction mixture is passed through an ion retardation column. This is a notable distinction over the prior art methods where this is a required step in order to remove ions and to neutralise the reaction mixture (e.g. as described by McConathy et al (2003 Appl Radiat Isotop; 58: 657-666), and in EP20172580029 (A)). As such, the method of the present invention is simplified over the prior art methods and as such is more suitable for automation.

In a preferred embodiment the method of the invention is carried out on an automated synthesis apparatus. By the term "automated synthesis apparatus" is meant an automated module based on the principle of unit operations as described by Satyamurthy et al (1999 Clin Positr Imag; 2(5): 233-253). The term 'unit operations" means that complex processes are reduced to a series of simple operations or reactions, which can be applied to a range of materials. Such automated synthesis apparatuses are preferred for the method of the present invention especially when a radiopharmaceutical composition is desired. They are commercially available from a range of suppliers (Satyamurthy *et al,* above), including: GE Healthcare; CTI Inc; Ion Beam Applications S.A. (Chemin du Cyclotron 3, B-1348 Louvain-La-Neuve, Belgium); Raytest (Germany) and Bioscan (USA).

A commercial automated synthesis apparatus also provides suitable containers for the liquid radioactive waste generated as a result of the radiopharmaceutical preparation. Automated synthesis apparatuses are not typically provided with radiation shielding, since they are designed to be employed in a suitably configured radioactive work cell. The radioactive work cell provides suitable radiation shielding to protect the operator from potential radiation dose, as well as ventilation to remove chemical and/or radioactive vapours. The automated synthesis apparatus preferably comprises a cassette. By the term "cassette" is meant a piece of apparatus designed to fit removably and interchangeably onto an automated synthesis apparatus, in such a way that mechanical movement of moving parts of the synthesizer controls the operation of the cassette from outside the cassette, i.e. externally. Suitable cassettes comprise a linear array of valves, each linked to a port where reagents or vials can be attached, by either needle puncture of an inverted septum-sealed vial, or by gas-tight, marrying joints. Each valve has a male-female joint which interfaces with a corresponding moving arm of the automated synthesis apparatus. External rotation of the arm thus controls the opening or closing of the valve when the cassette is attached to the automated synthesis apparatus. Additional moving parts of the automated synthesis apparatus are designed to clip onto syringe plunger tips, and thus raise or depress syringe barrels.

The cassette is versatile, typically having several positions where reagents can be attached, and several suitable for attachment of syringe vials of reagents or chromatography cartridges (e.g. for SPE). The cassette always comprises a reaction vessel. Such reaction vessels are preferably 0.5 to 10 mL, more preferably 0.5 to 5 mL and most preferably 0.5 to 4 mL in volume and are configured such that 3 or more ports of the cassette are connected thereto, to permit transfer of reagents or solvents from various ports on the cassette. Preferably the cassette has 15 to 40 valves in a linear array, most preferably 20 to 30, with 25 being especially preferred. The valves of the cassette are preferably each identical, and most preferably are 3-way valves. The cassettes are designed to be suitable for radiopharmaceutical manufacture and are therefore manufactured from materials which are of pharmaceutical grade and ideally also are resistant to radiolysis.

Preferred automated synthesis apparatuses for use with the present invention comprise a disposable or single use cassette which comprises all the reagents, reaction vessels and apparatus necessary to carry out the preparation of a given batch of radiofluorinated radiopharmaceutical. The cassette means that the automated synthesis apparatus has the flexibility to be capable of making a variety of different radiopharmaceuticals with minimal risk of cross-contamination, by simply changing the cassette. The cassette approach also has the advantages of: simplified set-up hence reduced risk of operator error; improved GMP (Good Manufacturing Practice) compliance; multi-tracer capability; rapid change between production runs; pre-run automated diagnostic checking of the cassette and reagents; automated barcode cross-check of chemical reagents *vs* the synthesis to be carried out; reagent traceability; single-use and hence no risk of cross-contamination, tamper and abuse resistance.

The following example serves to further illustrate the invention.

### Brief Description of the Examples

Example 1 describes a method to obtain the composition of the present invention.

### List of Abbreviations used in the Examples

- ATR: attenuated total reflectance
- DTGS: deuterated triglycine sulphate
- [¹⁸F]FACBC: 1-amino-3-[¹⁸F]fluorocyclobutane-1-carboxylic acid
- FT-IR: Fourier transform infrared
- K222: Kryptofix 222
- MeCN: acetonitrile
- MeOH: methanol
- QMA: quaternary methyl ammonium
- RCY: radiochemical yield
- SPE: solid-phase extraction
- TLC: thin layer chromatography
- UV: ultraviolet

### Examples

All reagents and solvents were purchased from Merck and used without further purification. The [¹⁸F]FACBC precursor, *Syn*-1-(N-(tert-butoxycarbonyl)amino)-3-[[(trifluoromethyl)sulfonyl]oxy]-cyclobutane-l-carboxylic acid ethyl ester was obtained from GE Healthcare. The Oasis HLB plus cartridge and the Sep-Pak cartridges: QMA light Plus (K₂CO₃ form), tC18 light, Alumina N light were purchased from Waters (Milford, MA, USA). A Capintec NaI ion chamber was used for all radioactive measurements (model CRC15R). Radio-thin layer chromatography (radio-TLC) was performed on a Packard instant imager using pre-coated plates of silica gel (Merck 60F₂₅₄).

### Example 1: Synthesis and Formulation of [¹⁸F]FACBC Composition of the Invention

No-carrier-added [¹⁸F]fluoride was produced via the ¹⁸O(p,n)¹⁸F nuclear reaction on a GE PETtrace 6 cyclotron (Norwegian Cyclotron Centre, Oslo). Irradiations were performed using a dual-beam, 30µA current on two equal Ag targets with HAVAR foils using 16.5 MeV protons. Each target contained 1.6 ml of ≥ 96% [¹⁸O]water (Marshall Isotopes). Subsequent to irradiation and delivery to a hotcell, each target was washed with 1.6 ml of [¹⁶O]water (Merck, water for GR analysis), giving approximately 2-5 Gbq in 3.2 ml of [¹⁶O]water.

All radiochemistry was performed on a commercially available GE FASTlab™ with a single-use cassette. Each cassette is built around a one-piece-moulded manifold with 25 three-way stopcocks, all made of polypropylene. Briefly, the cassette includes a 5 ml reactor (cyclic olefin copolymer), one 1 ml syringe and two 5 ml syringes, spikes for connection with five prefilled vials, one water bag (100 ml) as well as various SPE cartridges and filters. Fluid paths are controlled with nitrogen purging, vacuum and the three syringes. The fully automated system is designed for single-step fluorinations with cyclotron-produced [¹⁸F] fluoride. The FASTlab was programmed by the software package in a step-by-step time-dependent sequence of events such as moving the syringes, nitrogen purging, vacuum, and temperature regulation. Synthesis of [¹⁸F]FACBC followed the three general steps: (a) [¹⁸F]fluorination, (b) hydrolysis of protection groups and (c) SPE purification.

Vial A contained K₂₂₂ (156 µmol), K₂CO₃ (60.8 µmol) in 79.5% (v/v) MeCN_{(aq)} (1105 µl). Vial B contained 4M HCl. Vial C contained MeCN. Vial D contained precursor (123.5 µmol) in its dry form (stored below -5 °C until cassette assembly). Vial E contained 2 M NaOH (4.1 ml). The 30 ml product collection glass vial was filled with 200 mM citrate buffer (10 ml). Aqueous (¹⁸F]fluoride (1-1.5 ml, 100-200 Mbq) was passed through the QMA and into the ¹⁸O-H₂O recovery vial. The QMA was then flushed with MeCN and sent to waste. The trapped [¹⁸F]fluoride was eluted into the reactor using eluent from vial A (730 µl) and then concentrated to dryness by azeotropic distillation with acetonitrile (80 µl, vial C). Approximately 1.7 ml of MeCN was mixed with precursor in vial D from which 1.0 ml of the dissolved precursor (corresponds to 72.7 mmol precursor) was added to the reactor and heated for 3 min at 85°C. The reaction mixture was diluted with water and sent through the tC18 cartridge. Reactor was washed with water and sent through the tC18 cartridge. The labelled intermediate, fixed on the tC18 cartridge was washed with water, and then incubated with 2M NaOH (2.0 ml) for 5 min. The labelled intermediate (without the ester group) was eluted off the tCl 8 cartridge into the reactor using water. The BOC group was hydrolysed by adding 4M HCl (1.4 ml) and heating the reactor for 5 min at 60 °C. The reactor content with the crude [¹⁸F]FACBC was sent through the HLB and Alumina cartridges and into the 30 ml product vial. The HLB and Alumina cartridges were washed with water (9.1 ml total) and collected in the product vial. Finally, 2M NaOH (0.9 ml) and water (2.1 ml) was added to the product vial, giving the purified formulation of e^{8F}]FACBC with a total volume of 26 ml. Radiochemical purity was measured by radio-TLC using a mixture of MeCN:MeOH:H₂O:CH₃COOH (20:5:5:1) as the mobile phase. The radiochemical yield (RCY) was expressed as the amount of radioactivity in the [¹⁸F]FACBC fraction divided by the total used [¹⁸F]fluoride activity (decay corrected). Total synthesis time was 43 min.

## Claims

1. A pharmaceutical composition of ¹⁹F-FACBC **characterised in that** said composition:
(i) comprises 50-100 mM citrate buffer; and,
(ii) has a pH of 4.0-5.0
with the proviso that said composition does not comprise a sugar lactone or a sugar alcohol radiostabiliser.

2. The pharmaceutical composition as defined in Claim 1 comprising 60-90 mM citrate buffer.

3. The pharmaceutical composition as defined in either Claim 1 or Claim 2 comprising 75-85 mM citrate buffer.

4. The pharmaceutical composition as defined in any one of Claims 1-3 that has a pH of 4.1-4.5.

5. The pharmaceutical composition as defined in any one of Claims 1-4 that has an end of synthesis (EOS) radioactive concentration (RAC) of at least 1000 MBq/mL.

6. The pharmaceutical composition as defined in any one of Claims 1-5 which comprises not more than 150 µg/mL 1-amino-3-hydroxyl-cyclobutane-1-carboxylic acid (hydroxyl-ACBC).

7. The pharmaceutical composition as defined in any one of Claims 1-6 which comprises not more than 80 µg/mL hydroxyl-ACBC.

8. The pharmaceutical composition as defined in any one of Claims 1-7 which comprises not more than 0.15 µg/mL 1-amino-3-fluoro-cyclobutane-1-carboxylic acid (FACBC).

9. The pharmaceutical composition as defined in any one of Claims 1-8 which comprises not more than 0.10 µg/mL FACBC.

10. The pharmaceutical composition as defined in any one of Claims 1-9 which comprises not more than 2.0 µg/mL 1-amino-3-chloro-cyclobutane-1-carboxylic acid (chloro-ACBC).

11. The pharmaceutical composition as defined in any one of Claims 1-10 which comprises not more than 1.0 µg/mL chloro-ACBC.

12. A method to obtain a radiopharmaceutial composition wherein said composition is as defined in any one of Claims 1-11 and wherein said method comprises:
(i) reacting with a suitable source of [¹⁸F]fluoride a precursor compound of Formula I: 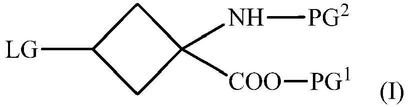 wherein:
LG is a leaving group;
PG¹ is a carboxy protecting group; and,
PG² is an amine protecting group;
to obtain a compound of Formula II: 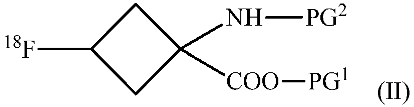 wherein PG¹ and PG² are as defined for Formula II;
(ii) reacting said compound of Formula II with a PG¹ deprotecting agent to obtain a compound of Formula III: 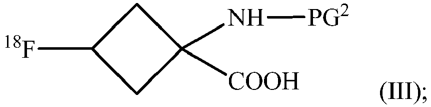 wherein PG² is as defined for Formula I;
(iii) reacting said compound of Formula III with a PG² deprotecting agent to obtain [¹⁸F]FACBC;
(iv) formulating said [¹⁸F]FACBC with citrate buffer to obtain said pharmaceutical composition.

13. The method as defined in Claim 12 wherein said [¹⁸F]-FACBC is trans-1-amino-3-[¹⁸F]-fluorocyclobutanecarboxylic acid (*anti*-[¹⁸F]-FACBC): 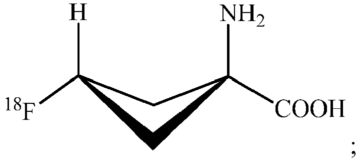 said compound of Formula I is a compound of Formula la: 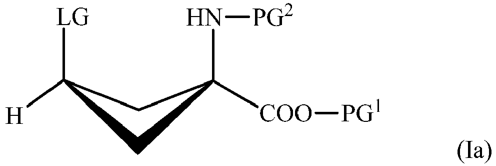 said compound of Formula II is a compound of Formula IIa: 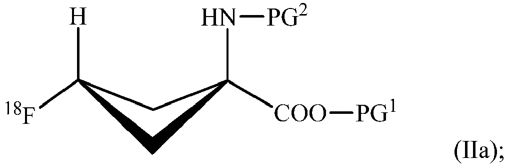 and,
said compound of Formula III is a compound of Formula IIIa: 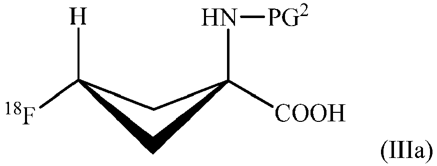 wherein PG¹ and PG² are as defined in Claim 12 for Formula I.

## Patentansprüche

1. Pharmazeutische ¹⁸F-FACBC-Zusammensetzung, **dadurch gekennzeichnet, dass** die Zusammensetzung:
(i) 50 bis 100 mM Citratpuffer umfasst und
(ii) einen pH-Wert von 4,0 bis 5,0 aufweist,
unter der Bedingung, dass die Zusammensetzung kein Zuckerlacton oder keinen Zuckeralkohol als Radiostabilisator umfasst.

2. Pharmazeutische Zusammensetzung nach Anspruch 1, die 60 bis 90 mM Citratpuffer umfasst.

3. Pharmazeutische Zusammensetzung nach Anspruch 1 oder Anspruch 2, die 75 bis 85 mM Citratpuffer umfasst.

4. Pharmazeutische Zusammensetzung nach einem der Ansprüche 1 bis 3, die einen pH-Wert von 4,1 bis 4,5 aufweist.

5. Pharmazeutische Zusammensetzung nach einem der Ansprüche 1 bis 4, die eine radioaktive Konzentration (radioactive concentration, RAC) zum Ende der Synthese (end of synthesis, EOS) von mindestens 1000 MBq/ml aufweist.

6. Pharmazeutische Zusammensetzung nach einem der Ansprüche 1 bis 5, die nicht mehr als 150 µg/ml 1-Amino-3-hydroxyl-cyclobutan-1-carbonsäure (Hydroxyl-ACBC) umfasst.

7. Pharmazeutische Zusammensetzung nach einem der Ansprüche 1 bis 6, die nicht mehr als 80 µg/ml Hydroxyl-ACBC umfasst.

8. Pharmazeutische Zusammensetzung nach einem der Ansprüche 1 bis 7, die nicht mehr als 0,15 µg/ml 1-Amino-3-fluor-cyclobutan-1-carbonsäure (FACBC) umfasst.

9. Pharmazeutische Zusammensetzung nach einem der Ansprüche 1 bis 8, die nicht mehr als 0,10 µg/ml FACBC umfasst.

10. Pharmazeutische Zusammensetzung nach einem der Ansprüche 1 bis 9, die nicht mehr als 2,0 µg/ml 1-Amino-3-chlor-cyclobutan-1-carbonsäure (Chlor-ACBC) umfasst.

11. Pharmazeutische Zusammensetzung nach einem der Ansprüche 1 bis 10, die nicht mehr als 1,0 µg/ml Chlor-ACBC umfasst.

12. Verfahren zum Erhalten einer radiopharmazeutischen Zusammensetzung, wobei die Zusammensetzung einem der Ansprüche 1 bis 11 entspricht und wobei das Verfahren umfasst:
(i) Reagierenlassen, mit einer geeigneten Quelle für [¹⁸F]-Fluorid, einer Vorstufenverbindung mit der Formel I: 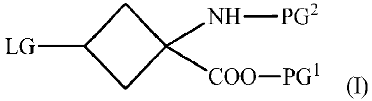 worin:
LG eine Fluchtgruppe ist;
PG¹ eine Carboxy-Schutzgruppe ist und
PG² eine Amin-Schutzgruppe ist;
zum Erhalten einer Verbindung mit der Formel II: 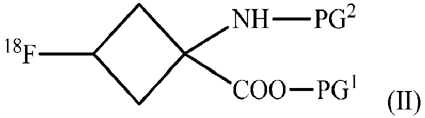 worin PG¹ und PG² wie für Formel II definiert sind;
(ii) Reagierenlassen der Verbindung mit der Formel II mit einem PG¹-Entschützungsmittel zum Erhalten einer Verbindung mit der Formel III: 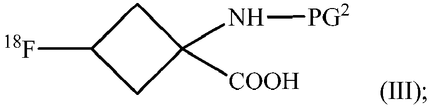 worin PG² wie für Formel I definiert ist;
(iii) Reagierenlassen der Verbindung mit der Formel III mit einem PG²-Entschützungsmittel zum Erhalten von [¹⁸F]FACBC;
(iv) Formulieren von [¹⁸F]FACBC mit Citratpuffer zum Erhalten der pharmazeutischen Zusammensetzung.

13. Verfahren nach Anspruch 12, wobei [¹⁸F]-FACBC trans-1-Amino-3-[¹⁸F]-fluorcyclobutancarbonsäure (*anti*-[¹⁸F]-FACBC) ist: 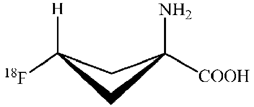 die Verbindung mit der Formel I eine Verbindung mit der Formel Ia ist: 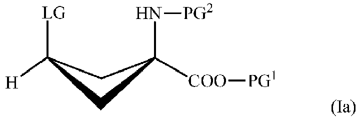 die Verbindung mit der Formel II eine Verbindung mit der Formel IIa ist: 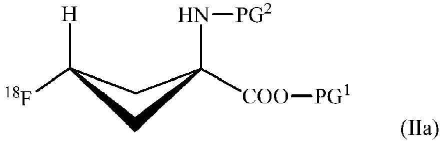 und
die Verbindung mit der Formel III eine Verbindung mit der Formel IIIa ist: 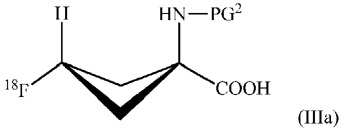 worin PG¹ und PG² wie in Anspruch 12 für Formel I definiert sind.

## Revendications

1. Composition pharmaceutique de ¹⁸F-FACBC **caractérisée en ce que** ladite composition :
(i) comprend un tampon citrate 50 à 100 mM ; et,
(ii) a un pH de 4,0 à 5,0
à condition que ladite composition ne comprenne pas une lactone de sucre ou un radiostabilisant polyalcool.

2. Composition pharmaceutique telle que définie dans la revendication 1 comprenant un tampon citrate 60 à 90 mM.

3. Composition pharmaceutique telle que définie dans la revendication 1 ou la revendication 2 comprenant un tampon citrate 75 à 85 mM.

4. Composition pharmaceutique telle que définie dans l'une quelconque des revendications 1 à 3 qui a un pH de 4,1 à 4,5.

5. Composition pharmaceutique telle que définie dans l'une quelconque des revendications 1 à 4 qui a une concentration radioactive (RAC) de fin de synthèse (EOS) d'au moins 1000 MBq/ml.

6. Composition pharmaceutique telle que définie dans l'une quelconque des revendications 1 à 5 qui ne comprend pas plus de 150 µg/ml d'acide 1-amino-3-hydroxyl-cyclobutane-1-carboxylique (hydroxyl-ACBC).

7. Composition pharmaceutique telle que définie dans l'une quelconque des revendications 1 à 6 qui ne comprend pas plus de 80 µg/ml d'hydroxyl-ACBC.

8. Composition pharmaceutique telle que définie dans l'une quelconque des revendications 1 à 7 qui ne comprend pas plus de 0,15 µg/ml d'acide 1-amino-3-fluoro-cyclobutane-1-carboxylique (FACBC).

9. Composition pharmaceutique telle que définie dans l'une quelconque des revendications 1 à 8 qui ne comprend pas plus de 0,10 µg/ml de FACBC.

10. Composition pharmaceutique telle que définie dans l'une quelconque des revendications 1 à 9 qui ne comprend pas plus de 2,0 µg/ml d'acide 1-amino-3-chloro-cyclobutane-1-carboxylique (chloro-ACBC).

11. Composition pharmaceutique telle que définie dans l'une quelconque des revendications 1 à 10 qui ne comprend pas plus de 1,0 µg/ml de chloro-ACBC.

12. Procédé permettant d'obtenir une composition radiopharmaceutique, dans lequel ladite composition est telle que définie dans l'une quelconque des revendications 1 à 11 et dans lequel ledit procédé comprend :
(i) la réaction avec une source appropriée de [¹⁸F]fluorure d'un composé précurseur de Formule I : 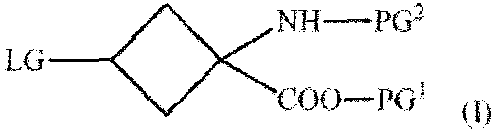 dans lequel :
LG est un groupe partant ;
PG¹ est un groupe carboxy protecteur ; et,
PG² est un groupe amine protecteur ;
pour obtenir un composé de Formule II : 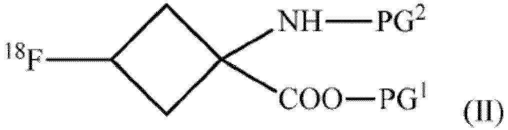 dans lequel PG¹ et PG² sont tels que définis pour la Formule II ;
(ii) la réaction dudit composé de Formule II avec un agent déprotecteur de PG¹ pour obtenir un composé de Formule III : 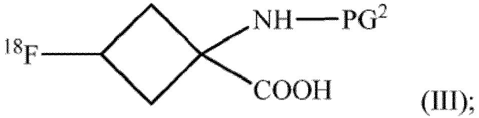 dans lequel PG² est tel que défini pour la Formule I ;
(iii) la réaction dudit composé de Formule III avec un agent déprotecteur de PG² pour obtenir [¹⁸F]FACBC ;
(iv) la formulation dudit [¹⁸F]FACBC avec un tampon citrate pour obtenir ladite composition pharmaceutique.

13. Procédé tel que défini dans la revendication 12 dans lequel ledit [¹⁸F]-FACBC est l'acide trans-1-amino-3-[¹⁸F]-fluorocyclobutanecarboxylique (*anti*-[¹⁸F]-FACBC) : 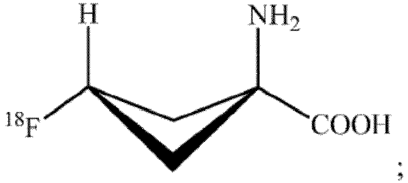 ledit composé de Formule I est un composé de Formule la : 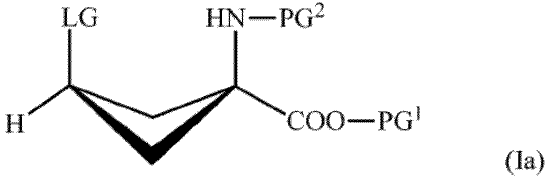 ledit composé de Formule II est un composé de Formule IIa : 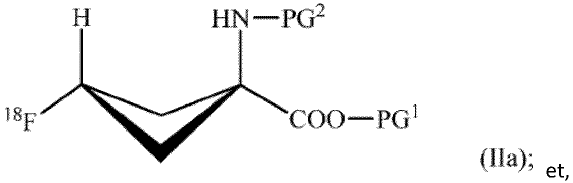 ledit composé de Formule III est un composé de Formule IIIa : 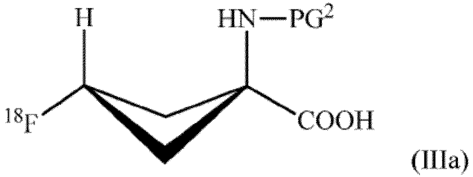 dans lequel PG¹ et PG² sont tels que définis dans la revendication 12 pour la Formule I.
